(19) Europäisches Patentamt
European Patent Office
Office européen des brevets

(11) EP 4 527 413 A1

(12) EUROPEAN PATENT APPLICATION
published in accordance with Art. 153(4) EPC

(43) Date of publication:
**26.03.2025 Bulletin 2025/13**

(21) Application number: **23879840.9**

(22) Date of filing: **18.10.2023**

(51) International Patent Classification (IPC):
***A61K 47/34*** *(2017.01)* ***A61K 47/32*** *(2006.01)*
***A61K 48/00*** *(2006.01)* ***A61P 17/02*** *(2006.01)*

(52) Cooperative Patent Classification (CPC):
**A61K 47/32; A61K 47/34; A61K 48/00; A61P 17/02**

(86) International application number:
**PCT/JP2023/037744**

(87) International publication number:
**WO 2024/085197 (25.04.2024 Gazette 2024/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.10.2022 JP 2022169353**

(71) Applicants:
- **The University of Tokyo**
  **Bunkyo-ku, Tokyo 113-8654 (JP)**
- **Gellycle Co., Ltd.**
  **Tokyo 113-0033 (JP)**

(72) Inventors:
- **SAKAI Takamasa**
  **Tokyo 113-8654 (JP)**
- **KURITA Masakazu**
  **Tokyo 113-8654 (JP)**
- **KATASHIMA Takuya**
  **Tokyo 113-8654 (JP)**
- **KATOH Motoi**
  **Tokyo 113-8654 (JP)**
- **ISHIKAWA Shohei**
  **Tokyo 113-8654 (JP)**
- **KAMATA Hiroyuki**
  **Tokyo 113-0033 (JP)**
- **MASUI Kosuke**
  **Tokyo 113-0033 (JP)**

(74) Representative: **Symbiosis IP Limited**
**The Innovation Centre**
**217 Portobello**
**Sheffield, South Yorkshire S1 4DP (GB)**

# (54) CARRIER MATERIAL FOR LOCALLY RELEASING PARTICULATE SUBSTANCE

(57) An object of the present invention is to provide a biocompatible carrier material capable of locally releasingparticles.

The present inventors have found that by using a non-gelling polymer formed by reversibly bonding a plurality of hydrophilic polymer units as a carrier material, it is possible to locally release particles to an affected part such as an ulcer surface, thereby enhancing gene transduction specificity and reducing off-target effects.

*FIG. 4*

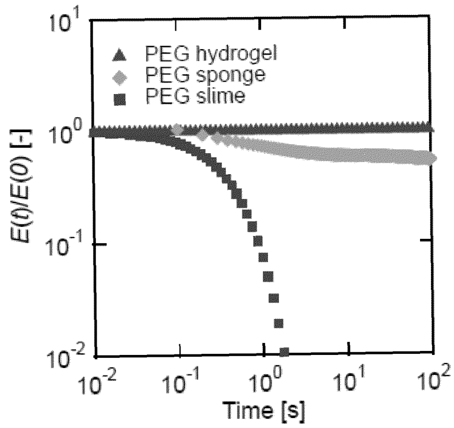


EP 4 527 413 A1

## Description

### Technical Field

The present invention relates to an associative carrier material for locally releasing particles, particularly, a carrier material suitable for the local release of adeno-associated virus (AAV).

### Background Art

Adeno-associated virus (AAV) is one of the most potent vectors for in vivo gene transduction. Starting with clinically proven systemic replacement therapies for lipoprotein lipase deficiency, spinal muscular atrophy, retinal dystrophy, hemophilia, and the like, recently, applications have also been greatly expanded to more localized morbidities.

With the improvement of the method for producing AAV vectors, the average doses of AAV vectors used in clinical trials are increasing. High doses can be used to achieve a stronger phenotype, but most of the AAV vectors reach the liver and can cause toxicity in the liver and elsewhere. In this context, control of specificity of gene expression, that is, minimization of off-target effects, has become one of the important development directions. To date, attempts have been made to minimize off-target effects by selection and engineering of AAV capsid tissue/cell tropism, use of tissue-specific promoters, and selection of the route of administration.

Non-healing wounds resulting from pressure ulcers, diabetic ulcers, and peripheral vascular insufficiency are of great concern in societies with progressively aging populations, and AAV-based in vivo gene transduction is expected as one of the most potent therapies. In particular, recently, in vivo induction of reprogramming of wound-resident mesenchymal cells into epithelial cells utilizing AAV has enabled de novo epithelialization from the surface of ulcers, which may lead to the development of innovative therapies.

### Citation List

### Non Patent Literature

Non Patent Literature 1: Kurita, M., Izpisua Belmonte, J. C., Suzuki, K., Okazaki, M., J Dermatol Sci 2019;95:8-12. Non Patent Literature 2: Kurita, M., Araoka, T., Hishida, T., et al., Nature 2018;561:243-247.

### Summary of Invention

### Technical Problem

Therefore, an object of the present invention is to provide a biocompatible carrier material capable of locally releasing particles such as adeno-associated virus on the surface of a skin ulcer or the like.

### Solution to Problem

As a result of intensive studies to solve the above problems, the present inventors have found that by using a non-gelling polymer formed by reversibly bonding a plurality of hydrophilic polymer units as a carrier material, it is possible to locally release particles to an affected part such as an ulcer surface, thereby enhancing gene transduction specificity and reducing off-target effects, and have completed the present invention.

That is, in one aspect, the present invention provides:

<1> a carrier material for locally releasing particles, wherein the carrier material has a finite zero-shear viscosity higher than 500 Pa · s, contains a non-gelling polymer formed by reversibly bonding a plurality of hydrophilic polymer units, and contains a solvent, the polymer units include a first branched polymer unit having a total of two or more boronic acid-containing groups on side chains or terminals, and a second branched polymer unit having a total of two or more diol groups on side chains or terminals, and a polymer concentration (c) in the carrier material is greater than or equal to an overlapping concentration (c*) of the polymer units;
<2> the carrier material according to <1>, wherein the particles have a diameter in the range of 10 to 100 nm;
<3> the carrier material according to <1>, wherein the particles include an adeno-associated virus (AAV);
<4> the carrier material according to <1>, wherein the polymer unit has a polyethylene glycol backbone or a polyvinyl backbone;
<5> the carrier material according to <1>, wherein the first branched polymer unit is di-, tri-, or tetra-branched polyethylene glycol;

<6> the carrier material according to <1>, wherein the second branched polymer unit is di-, tri-, or tetra-branched polyethylene glycol, or di-branched polyvinyl alcohol;

<7> the carrier material according to <1>, wherein the first branched polymer unit has a molecular weight (Mw) of $5 \times 10^3$ to $1 \times 10^5$;

<8> the carrier material according to <1>, wherein the boronic acid-containing group is an arylboronic acid optionally substituted with a halogen atom;

<9> the carrier material according to <1>, wherein the diol group has a ring-opened structure of a sugar derivative;

<10> the carrier material according to <1>, further comprising a third polymer unit having a total of two or more boronic acid-containing groups or diol groups on side chains or terminals;

<11> The carrier material according to <1>, wherein an osmotic pressure of the carrier material is less than an osmotic pressure $\Pi_0$ (Pa) expressed by the following expression:

[Expression 1]

$$\Pi_0 = \frac{M}{cRT}\{1 + c/c^* + 0.25(c/c^*)^2\}$$

wherein c represents a polymer concentration (g/L), c* represents an overlapping concentration of the polymer units, R represents gas constant, T represents an absolute temperature, and M is a weight average molecular weight of the polymer units;

<12> the carrier material according to <1>, wherein the carrier material has an ability to release the particles in vivo along with elution of the carrier material, and a dissolution rate L and a release rate M at that time satisfy a relationship of:

[Expression 2]

$$L = A \times M$$

wherein A is in a range of $0.9 < A < 1.1$; and

<13> the carrier material according to <1>, wherein an application of the locally releasing is a gene therapy for a skin ulcer.

In another aspect, the present invention provides:

<14> a composition containing the carrier material according to any one of <1> to <13> and the particles stored in the carrier material;

<15> a treatment method including applying the composition according to <14> to an affected part;

<16> a kit for forming the carrier material according to any one of <1> to <13>, including at least a composition A containing a first branched polymer unit having a total of two or more boronic acid-containing groups on side chains or terminals, and a composition B containing a second branched polymer unit having a total of two or more diol groups on side chains or terminals, wherein the carrier material has a finite zero-shear viscosity of greater than 500 Pa · s, and a polymer content (c) in the carrier material is greater than or equal to an overlapping concentration (c*) of the polymer units; and

<17> the kit according to <16>, further including a polymer solution C containing a third polymer unit having a total of two or more boronic acid-containing groups or diol groups on side chains or terminals.

## Advantageous Effects of Invention

According to the present invention, it is possible to provide a biocompatible carrier material capable of locally releasing particles such as adeno-associated virus on the surface of a skin ulcer or the like. As a result, the application to the gene therapy for skin ulcers and the like can be more safely performed.

## Brief Description of Drawings

Fig. 1 is a schematic diagram showing a structure of a carrier material (PEG slime) of the present invention used in Examples.

Fig. 2 is a schematic diagram showing structures of carrier materials (PEG hydrogel and PEG sponge) of Comparative Example used in Examples.

Fig. 3 is images showing viscosity of each carrier material used in Examples.

Fig. 4 is a graph showing a temporal change in Young's modulus for each carrier material used in Examples.

Fig. 5 is confocal microscope images showing an internal network structure for each carrier material used in Examples.

Fig. 6 is a graph plotting dissolution rates and release rates obtained for each carrier material used in Examples. Closed symbols indicate release rates (left axis), and open symbols indicate dissolution rates (right axis).

Fig. 7 is a graph plotting the release rates obtained in Fig. 6 against the dissolution rates.

Fig. 8 is a schematic diagram showing an experimental procedure of AAV gene transduction into a skin ulcer.

Fig. 9 is fluorescence microscopic images of the surface of ulcers when AAV gene transduction is performed using each carrier material.

Fig. 10 is a graph showing the numbers of GFP-positive cells expressed in the surface and deep layer parts when AAV gene transduction was performed using each carrier material.

Fig. 11 is stereomicroscope images and fluorescence microscope images of the surface of ulcers when superficial layer-specific AAV gene transduction was performed using PEG slime and PBS.

Fig. 12 is a graph showing the numbers of GFP-positive cells expressed in the superficial and deep layer parts when superficial layer-specific AAV gene transduction was performed using each carrier material.

Fig. 13 is fluorescence microscopic images of liver tissues when AAV gene transduction was performed using PEG slime and PBS.

Fig. 14 is a graph showing AAV titers in the liver.

Fig. 15 is a schematic diagram of an experimental procedure for evaluating the dispersion behavior of nanoparticles on the surface of a skin ulcer.

Fig. 16 is a graph showing the numbers of particles on the surface when superficial layer-specific AAV gene transduction was performed using each carrier material.

Fig. 17 is a schematic diagram of a local infection mechanism using PEG carriers (the upper figure a shows the case of PEG hydrogel and PEG sponge, and the lower figure b shows the case of PEG slime).

## Description of Embodiments

Hereinafter, embodiments of the present invention will be described. The scope of the present invention is not bound by these descriptions, and other than the following exemplifications, it is possible to appropriately change and implement within a range not to impair the gist of the present invention.

### 1. Carrier material of present invention

The carrier material of the present invention is a carrier material for locally releasing particles, wherein the carrier material has a finite zero-shear viscosity higher than 500 Pa · s; contains a non-gelling polymer formed by reversibly bonding a plurality of hydrophilic polymer units; and contains a solvent; the polymer units include a first branched polymer unit having a total of two or more boronic acid-containing groups on side chains or terminals, and a second branched polymer unit having a total of two or more diol groups on side chains or terminals; and a polymer concentration (c) in the carrier material is greater than or equal to an overlapping concentration (c*) of the polymer units.

Hereinafter, the polymer units constituting the carrier material of the present invention and the characteristics of the carrier material will be described in more detail.

#### (1-1) Polymer unit

The polymer units constituting the carrier material of the present invention can form a non-gel carrier material by being connected to each other, and more specifically, in the final carrier material, the polymer units are a branched polymer that can form an associate having a network structure, particularly a three-dimensional network structure by being connected through a reversible bond by an equilibrium reaction. Such polymer units are preferably hydrophilic polymers. As the hydrophilic polymer, a polymer having affinity for water known in the art can be used, but is preferably a biocompatible polymer having a polyalkylene glycol backbone or a polyvinyl backbone.

Examples of the polymer having a polyalkylene glycol backbone preferably include polymer species having a plurality of branches of a polyethylene glycol backbone, and particularly, di-, tri-, or tetra-branched polyethylene glycol is preferable. A carrier material composed of a tetra-branched polyethylene glycol backbone is generally known as a Tetra-PEG carrier material, and it is known that a network structure network is constructed by an AB-type cross-end coupling reaction between tetra-branched polymers each having two or more types of functional groups capable of reacting with

each other at the terminals (Matsunaga et al., Macromolecules, Vol. 42, No. 4, pp. 1344-1351, 2009). In addition, the Tetra-PEG carrier material can be easily prepared in situ by simple two-liquid mixing of each polymer solution, and it is also possible to control the carrier materialization time by adjusting the pH and ionic strength during preparation of the carrier material. It is known that a carrier material having excellent physical properties can also be prepared from a three-branched polyethylene glycol (Fujiyabu et al., Science Advances, Vol. 8, eabk0010, 2022). Moreover, since these carrier materials contain PEG as a main component, they are also excellent in biocompatibility.

In addition, examples of the hydrophilic polymer having a polyvinyl backbone include polyalkyl methacrylates such as polymethyl methacrylate, polyacrylates, polyvinyl alcohols, poly N-alkyl acrylamides, and polyacrylamides. The hydrophilic polymer having a polyvinyl backbone is preferably di-branched, and for example, di-branched polyvinyl alcohol is suitable.

The hydrophilic polymer has a weight average molecular weight (Mw) in the range of $5 \times 10^3$ to $1 \times 10^5$, preferably in the range of $5 \times 10^3$ to $5 \times 10^4$, and more preferably $1 \times 10^4$ to $5 \times 10^4$.

The hydrophilic polymer used in the present invention is a combination of a first branched polymer unit having a total of two or more boronic acid-containing groups on side chains or terminals and a second branched polymer unit having a total of two or more diol groups on side chains or terminals. Here, the total of the boronic acid-containing groups and the diol groups is preferably 5 or more. It is more preferable that these functional groups are present at terminals. For example, when the first polymer unit has a di-, tri-, or tetra-branched structure, it preferably has a boronic acid-containing group at each branch end, and when the second polymer unit has a di-, tri-, or tetra-branched structure, it preferably has a diol group at each branch end.

When the polymer unit has such a boronic acid-containing group and a diol group, as exemplified in the following equilibrium reaction scheme, the boronic acid site and an OH group of a polyol chemically react to obtain the carrier material of the present invention having a structure in which polymer units are reversibly bonded to each other. The specific structures of the boronic acid-containing group and the diol group shown in the reaction scheme are merely examples and are not limited thereto, and other types of boronic acid-containing groups and diol groups may be used as described later.

[Chemical 1]

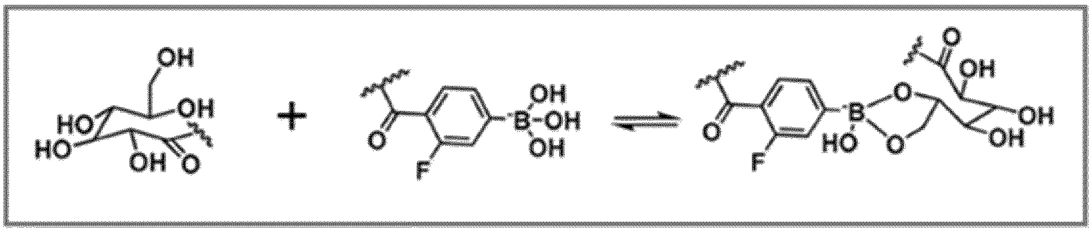

diol group boronic acid-containing group

The boronic acid-containing group present in the first polymer unit is not particularly limited as long as it has a structure having a boronic acid, and can be, for example, an arylboronic acid, preferably an arylboronic acid optionally substituted with a halogen atom. As the arylboronic acid, phenylboronic acid is preferable. The boronic acid-containing groups may be the same or different, but are preferably the same. When the functional groups are the same, reactivity with the diol group becomes uniform, and a carrier material having a uniform structure is easily obtained.

The diol group present in the second polymer unit is not particularly limited as long as it is a functional group having two or more hydroxyl groups (OH groups), but can be a sugar alcohol having a structure derived from sugar, preferably a sugar alcohol having a ring-opened structure of a sugar derivative. Such a sugar may be any of a monosaccharide, a disaccharide, and a polysaccharide, but can typically be a monosaccharide such as glucose or fructose. In addition, the diol group may be an aromatic polyol group or an aliphatic polyol group, and may be a diol group in which one or more carbons in the molecule are substituted with a hetero atom. The diol groups may be the same or different, but are preferably the same. When the functional groups are the same, reactivity with the boronic acid-containing group becomes uniform, and a carrier material having a uniform structure is easily obtained.

Preferably, the first branched polymer unit has a molecular weight (Mw) of $5 \times 10^3$ to $1 \times 10^5$ in weight average molecular weight. Similarly, the second branched polymer unit has a molecular weight (Mw) of $5 \times 10^3$ to $1 \times 10^5$.

Preferred non-limiting specific examples of the first polymer unit include, for example, a compound represented by the following formula (I) having four branches of a polyethylene glycol backbone and having a boronic acid-containing group at each terminal.

[Chemical 1]

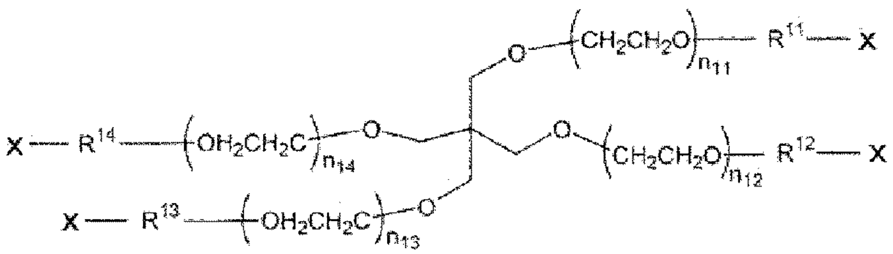

(I)

In the formula (I), X is a boronic acid-containing group, and in a preferred embodiment, X can be a fluorophenylboronic acid having the following structure (in the partial structure, the wavy line portion is a connection portion to $R^{11}$ to $R^{14}$.) .

[Chemical 1]

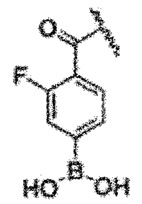

PEG-FPBA

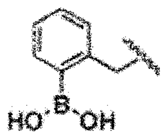

PEG-APBA

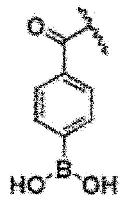

PEG-PBA

、 、 、

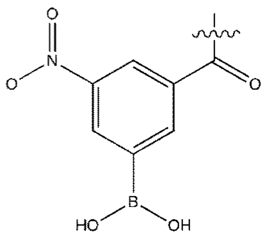

、

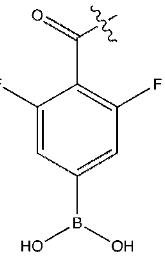

、

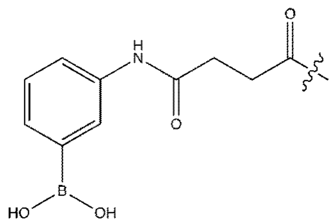

In the formula (I), $n_{11}$ to $n_{14}$ may be the same or different. As the values of $n_{11}$ to $n_{14}$ are closer to each other, a uniform three-dimensional structure can be obtained, and the strength becomes higher. Therefore, in order to obtain a high-strength carrier material, $n_{11}$ to $n_{14}$ are preferably the same. When the values of $n_{11}$ to $n_{14}$ are too high, the strength of the carrier material becomes weak, and when the values of $n_{11}$ to $n_{14}$ are too low, the carrier material is hardly formed due to steric hindrance of the compound. Therefore, examples of $n_{11}$ to $n_{14}$ include integer values of 20 to 570, preferably 20 to 290, and more preferably 50 to 290.

In the formula (I), $R^{11}$ to $R^{14}$ are the same or different, and each is a linker moiety connecting a functional group and a core portion. $R^{11}$ to $R^{14}$ may be the same or different, but are preferably the same in order to produce a high-strength carrier material having a uniform three-dimensional structure. $R^{11}$ to $R^{14}$ each represent a direct bond, a $C_1$-$C_7$ alkylene group, a $C_2$-$C_7$ alkenylene group, $-NH-R^{15}-$, $-CO-R^{15}-$, $-R^{16}-O-R^{17}-$, $-R^{16}-NH-R^{17}-$, $-R^{16}-CO_2-R^{17}-$, $-R^{16}-CO_2-NH-R^{17}-$, $-R^{16}-CO-R^{17}-$, or $-R^{16}-CO-NH-R^{17}-$. Here, $R^{15}$ represents a $C_1$-$C_7$ alkylene group. $R^{16}$ represents a $C_1$-$C_3$ alkylene group. $R^{17}$ represents a $C_1$-$C_5$ alkylene group.

Here, the "$C_1$-$C_7$ alkylene group" means an alkylene group having 1 or more and 7 or less carbon atoms which optionally have a branch, and means a linear $C_1$-$C_7$ alkylene group or a $C_2$-$C_7$ alkylene group having one or more branches (the number of carbon atoms including branches is 2 or more and 7 or less). Examples of the $C_1$-$C_7$ alkylene group are a methylene group, an ethylene group, a propylene group, and a butylene group. Examples of the $C_1$-$C_7$ alkylene groups include $-CH_2-$, $-(CH_2)_2-$, $-(CH_2)_3-$, $-CH(CH_3)-$, $-(CH_2)_3-$, $-(CH(CH_3))_2-$, $-(CH_2)_2-CH(CH_3)-$, $-(CH_2)_3-CH(CH_3)-$, $-(CH_2)_2-CH(C_2H_5)-$, $-(CH_2)_6-$, $-(CH_2)_2-C(C_2H_5)_2-$, and $-(CH_2)_3C(CH_3)_2CH_2-$.

The "$C_2$-$C_7$ alkenylene group" is a linear or branched alkenylene group having 2 to 7 carbon atoms and one or more double bonds in the chain, and examples thereof include a divalent group having a double bond formed by excluding 2 to 5 hydrogen atoms of adjacent carbon atoms from the alkylene group.

On the other hand, preferred non-limiting specific examples of the second polymer unit include, for example, a compound represented by the following formula (II) having four branches of a polyethylene glycol backbone and having a diol group at each terminal.

[Chemical 3]

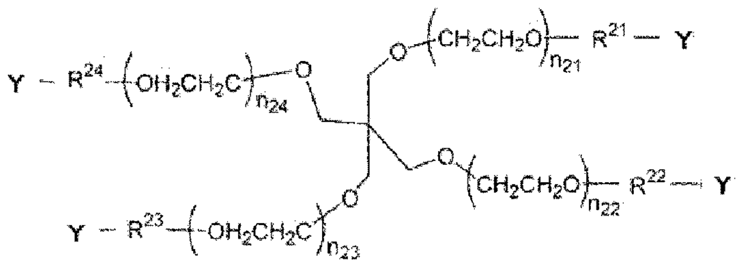

(II)

In the formula (I), Y is a diol group, and in a preferred embodiment, Y can be a polyol having a ring-opened structure of a glucose derivative having the following structure (in the partial structure, the wavy line portion is a connection portion to $R^{21}$ to $R^{24}$.).

[Chemical 4]

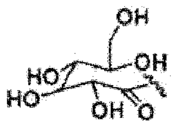

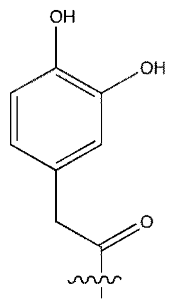

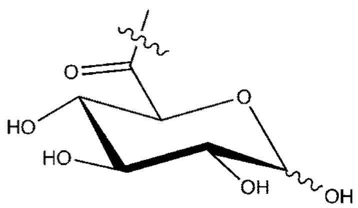

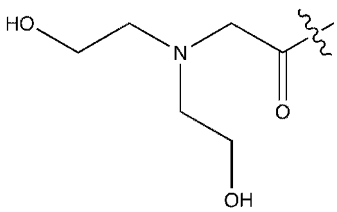

In the formula (II), $n_{21}$ to $n_{24}$ may be the same or different. As the values of $n_{21}$ to $n_{24}$ are closer to each other, the carrier material can have a uniform three-dimensional structure, resulting in high strength, and thus it is preferable, and it is preferable that the values are the same. When the values of $n_{21}$ to $n_{24}$ are too high, the strength of the carrier material becomes weak, and when the values of $n_{21}$ to $n_{24}$ are too low, the carrier material is hardly formed due to steric hindrance of the compound. Therefore, examples of $n_{21}$ to $n_{24}$ include integer values of 20 to 570, preferably 20 to 290, and more preferably 50 to 290.

In the formula (II), $R^{21}$ to $R^{24}$ are linker moieties connecting a functional group and a core portion. $R^{21}$ to $R^{24}$ may be the same or different, but are preferably the same in order to produce a high-strength carrier material having a uniform three-dimensional structure. In the formula (II), $R^{21}$ to $R^{24}$ are the same or different and each represent a direct bond, a $C_1$-$C_7$ alkylene group, a $C_2$-$C_7$ alkenylene group, -NH-$R^{25}$-, -CO-$R^{25}$-, -$R^{26}$-O-$R^{27}$-, -$R^{26}$-NH-$R^{27}$-, -$R^{26}$-$CO_2$-$R^{27}$-, -$R^{26}$-$CO_2$-NH-$R^{17}$-, -$R^{26}$-CO-$R^{27}$-, or-$R^{26}$-CO-NH-$R^{27}$-. Here, $R^{25}$ represents a $C_1$-$C_7$ alkylene group. $R^{26}$ represents a $C_1$-$C_3$ alkylene group. $R^{27}$ represents a $C_1$-$C_5$ alkylene group.

In the present specification, the alkylene group and the alkenylene group optionally have one or more arbitrary substituents. Examples of the substituent include, but are not limited to, alkoxy groups, halogen atoms (may be any of a fluorine atom, a chlorine atom, a bromine atom, and an iodine atom), amino groups, mono- or di-substituted amino groups, substituted silyl groups, an acyl group, and an aryl group. When the alkyl group has two or more substituents, they may be the same or different. The same applies to alkyl moieties of other substituents including alkyl moieties (for example, alkoxy groups and arylalkyl groups).

In addition, in the present specification, when a certain functional group is defined as "optionally having a substituent", the type of the substituent, the substitution position, and the number of substituents are not particularly limited, and when having two or more substituents, they may be the same or different. Examples of the substituent include, but are not limited to, alkyl groups, alkoxy groups, hydroxyl groups, carboxyl groups, halogen atoms, sulfo groups, amino groups, alkoxycarbonyl groups, and oxo groups. Substituents may be further present in these substituents.
In the present invention, in addition to the first and second polymer units, a third polymer unit having a total of two or more boronic acid-containing groups or diol groups on side chains or terminals can be further used. The third polymer unit is preferably a hydrophilic polymer, and the above description for the first and second polymer units shall apply equally to the structure of the third polymer unit.

(1-2) Physical properties and the like of carrier material

As described above, the carrier material of the present invention has a network structure formed of a first branched polymer unit and a second branched polymer unit, but as described above, the carrier material itself formed has characteristics that a polymer concentration (polymer content) (c) thereof is greater than or equal to an overlapping concentration (c*) of the polymer units ($c^* \leq c$). Preferably, the polymer concentration (c) can be 1.5 times or more of c* ($1.5c^* \leq c$), and more preferably 2 times or more of c* ($2c^* \leq c$) .
Furthermore, the carrier material of the present invention has a characteristic of a finite value of zero-shear viscosity of 500 Pa · s or more. The finite value of zero-shear viscosity can be preferably 1000 Pa · s or more, and more preferably 1200 Pa · s or more. The "zero-shear viscosity" means the viscosity when the shear rate reaches zero. A method of calculating the zero-shear viscosity can be determined by a generally known method. For example, as described in Polymer Physics (written by M. Rubinstein, R. Colby), it can be determined by dynamic viscoelasticity measurement using the slope of the loss modulus in the low frequency region with respect to the angular frequency. This makes it possible to reduce the osmotic pressure of a desired polymer solution to a desired range while maintaining physical properties such as consistency.
Here, the "overlapping concentration" (also called "overlap concentration".) is a concentration at which polymers in a solvent start to spatially come into contact with each other, and in general, the overlap concentration c* is represented by the following expression:

[Expression 3]

$$c^* = 3M_w / (4\pi \cdot \alpha \cdot N_A \cdot R_g^3)$$

wherein $M_w$ is the weight average molecular weight of the polymer; $\alpha$ is the specific gravity of the solvent; $N_A$ is the Avogadro constant; $R_g$ is the radius of gyration of the polymer.
For a method of calculating the overlap concentration c*, for example, Polymer Physics (written by M. Rubinstein, R. Colby) can be referred to. Specifically, for example, the overlap concentration can be determined by measuring the viscosity of a dilute solution using the Flory-Fox Equation.
The carrier material of the present invention contains a solvent and has a polymer concentration (polymer content) of 80 g/L or less, preferably 60 g/L or less, and more preferably 40 g/L or less. The lower limit value of the polymer concentration is not particularly limited, but the lower limit value is preferably 10 g/L or more from the viewpoint of obtaining desired physical properties such as consistency. The numerical range defines the polymer concentration effective for the carrier material of the present invention to reduce the osmotic pressure of the polymer solution by introduction of the associative site. The solvent in the carrier material is preferably water or a mixed solvent of water and an organic solvent.
Furthermore, the carrier material of the present invention preferably has an osmotic pressure in the range of 100 to 20,000 Pa, and more preferably in the range of 10 to 8,500 Pa. As described above, although the carrier material of the present invention is a polymer aqueous solution, the carrier material achieves a low osmotic pressure as compared with a polymer solution having a main chain having the same composition. Here, the osmotic pressure is represented by the following expression:

[Expression 4]

$$\Pi = 1.71c^{2.31}$$

wherein $\Pi$ represents osmotic pressure (Pa), and c represents the polymer content (g/L) in the carrier material.
Moreover, the osmotic pressure $\Pi$ of the carrier material of the present invention is preferably smaller than the

osmotic pressure $\Pi_0$ (Pa) expressed by the following expression ($\Pi < \Pi_0$). The osmotic pressure $\Pi_0$ is a theoretical value of the osmotic pressure in the case of the above-described "polymer having a main chain of the same composition".

[Expression 5]

$$\Pi_0 = \frac{M}{cRT}\{1 + c/c^* + 0.25(c/c^*)^2\}$$

wherein c represents polymer concentration (g/L), c* represents the overlapping concentration of the polymer units, R represents gas constant, T represents absolute temperature, and M is the weight average molecular weight of the polymer units.

As the solvent contained in the carrier material of the present invention, any solvent can be used as long as the associate formed by the polymer units is dissolved, but typically, water or an organic solvent can be used. As the organic solvent, alcohols such as ethanol and polar solvents such as DMSO can be used. Preferably, the solvent is water.

The carrier material of the present invention can typically be produced by mixing a raw material containing a first polymer unit and a raw material containing a second polymer unit. When a solution containing a polymer unit is used as a raw material, the concentration, addition rate, mixing rate, and mixing ratio of each solution are not particularly limited, and can be appropriately adjusted by those skilled in the art. As described above, water, alcohols such as ethanol, DMSO, and the like can be used as the solvent of the solution. When the solution is an aqueous solution, an appropriate pH buffer such as a phosphate buffer can be used. Preferably, the carrier material of the present invention can be prepared by using a kit described later.

The particles locally released by the carrier material of the present invention are not particularly limited as long as they can be retained in the carrier material, but preferably has a diameter in the range of 10 to 100 nm. Typical examples of such particles include adeno-associated virus (AAV). As described above, according to the carrier material of the present invention, AAV can be locally released to an affected part such as an ulcer surface, thereby enhancing gene transduction specificity and reducing off-target effects.

The mechanism of such local release is that the particles can be released in vivo as the carrier material of the present invention is eluted. Preferably, the dissolution rate L and the release rate M at that time satisfy the following relationship:

$$L = A \times M$$

wherein A is in the range of $0.9 < A < 1.1$.

(1-3) Kit

In another aspect, the present invention relates to a kit for forming the carrier material.

Such a kit includes at least a composition A containing a first branched polymer unit having a total of two or more boronic acid-containing groups on side chains or terminals, and a composition B containing a second branched polymer unit having a total of two or more diol groups on side chains or terminals. By mixing these, the carrier material of the present invention having a network structure composed of a plurality of polymer units can be obtained in-situ. The types and the like of the first and second polymer units are as described above.

The compositions A and B containing polymer units may be in a solution state or in a powder state, but are preferably in solution. In some cases, one of the compositions A and B can be a solution and the other can be a powder. Here, for convenience, the compositions A and B in a solution state are referred to as polymer solutions A and B, respectively. The solvent in the polymer solutions A and B is water, but in some cases, can be a mixed solvent containing alcohols such as ethanol and other organic solvents. Preferably, the polymer solutions A and B are aqueous solutions using water as a sole solvent. The volumes of the polymer solutions A and B can be appropriately adjusted according to the area of the affected part or the like to which they are applied, the complexity of the structure, and the like, but are typically in the range of 0.1 to 20 ml each, and preferably 1 to 10 ml.

The pH of the polymer solutions A and B is typically in the range of 4 to 10, preferably in the range of 5 to 8, and more preferably 7 to 8. For adjusting the pH of the polymer solutions A and B, a pH buffering agent known in the art can be used. For example, the pH can be adjusted to the above range by using a citric acid-phosphate buffer (CPB) and changing the mixing ratio of citric acid and disodium hydrogen phosphate.

As a means for mixing the polymer solutions A and B, for example, a two-liquid mixing syringe as disclosed in WO2007/083522 A can be used. The temperature of the two liquids at the time of mixing is not particularly limited as long as

the polymer units are each dissolved and the respective liquids have fluidity. For example, the temperatures of the two liquids may be different, but it is preferable that the temperatures are the same because the two liquids are easily mixed.

The kit of the present invention can further include a polymer solution C containing a third polymer unit having a total of two or more boronic acid-containing groups or diol groups on side chains or terminals.

The kit of the present invention can include a container storing the compositions A and B. For example, when the compositions A and B are in a solution state, an instrument such as a sprayer storing the solutions A and B can be used. As the sprayer, a sprayer known in the art can be appropriately used, and a medical sprayer is preferable. In that case, one aspect of the present invention can be a medical device, preferably a sprayer, storing the compositions A and B.

## Examples

The present invention is described in further detail below using examples, but the present invention is not limited to these examples.

### 1. Preparation of Carrier Material

Tetra-PEG-GDL having diol groups (ring-opened structure of gluconolactone) at the terminal of tetra-branched polyethylene glycol and Tetra-PEG-FPBA having a fluorophenylboronic acid group at the terminal were synthesized as polymer units constituting a carrier material.

The following raw materials were used. Tetra-PEG-$NH_2$ (those having molecular weights Mw of 5 k, 10 k, and 20 k were used; Yuka Sangyo Co., Ltd.); 4-carboxy-3 fluorophenylboronic acid (FPBA) (FUJIFILM Wako Pure Chemical Corporation); 4-(4,6-dimethoxy-1,3,5-triazin-2-yl)-4-methylmorpholinium chloride (DMT-MM) (FUJIFILM Wako Pure Chemical Corporation); and Glucono-δ-lactone (GDL) (Tokyo Chemical Industry Co., Ltd.).

### Synthesis of Tetra-PEG-GDL

Tetra-PEG-$NH_2$ having an amino group at the terminal was dissolved in methanol at a concentration of 50 mg/mL, 10 times gluconolactone and 20 times triethylamine in molar ratio to the terminal amino group of Tetra-PEG-$NH_2$ were added, and the mixture was stirred at 35°C for 3 days. The reaction solution was transferred to a dialysis membrane (MWCO for 20 k: 6-8,000, MWCO for 10 k, 5 k: 3,500), dialyzed against methanol for 2 days and water for 2 days, passed through a 0.45 um syringe filter, and then freeze-dried, and collected as a powder. The completion of the synthesis was confirmed by $^1$H-NMR.

### Synthesis of Tetra-PEG-FPBA

Tetra-PEG-$NH_2$ was dissolved in methanol at a concentration of 50 mg/mL, 5 times FPBA and 10 times DMT-MM in molar ratio to the terminal amino group of Tetra-PEG-$NH_2$ were added, and the mixture was stirred at room temperature overnight. The reaction solution was transferred to a dialysis membrane (MWCO for 20 k: 6-8,000, MWCO for 10 k, 5 k: 3,500), and dialyzed for half a day against an aqueous hydrochloric acid solution (10 mM), half a day against an aqueous sodium hydroxide solution (10 mM), half a day against a phosphate buffer (pH 7.4, 10 mM), one day against saline (100 mM), and finally one day against pure water, and passed through a 0.45 μm syringe filter, then freeze-dried, and collected as a powder. The completion of the synthesis was confirmed by $^1$H-NMR.

Using di-branched PEG and tri-branched PEG as raw materials, various polymer units having a GDL terminal and FPBA were prepared in the same manner.

### Synthesis of Carrier Material

A solution containing the obtained polymer unit was prepared under the following conditions, and these polymer solutions were mixed to synthesize a carrier material.

[Polymer solution A]

Concentration: 2-10 wt% Tetra-PEG-GDL
pH: 7.4
[Polymer solution B]
Concentration: 2-10 wt% Tetra-PEG-FPBA

pH: 7.4

2. Evaluation of Osmotic Pressure

The osmotic pressure of the carrier material obtained above was measured. The results when the molecular weight of the monomer unit was 10 k and 40 k are shown in Figs. 1 and 2, respectively. With the increase in the polymer content c, the osmotic pressure $\Pi$ increased. A solid line in Fig. 1 shows a regression curve of these data. All data were on the same curve regardless of pH, the number of branches, and molecular weight. This regression curve can be expressed by the following expression:

[Expression 6]

$$\Pi = 1.71c^{2.31}$$

wherein $\Pi$ represents osmotic pressure (Pa), and c represents the polymer content (g/L) in the carrier material.;

Figs. 1 and 2 also show results of osmotic pressure of a normal polymer solution having no associative functional group (di-, tri-, or tetra-branched aqueous PEG solution terminated with carboxyl groups) as a comparative example. Also in these cases, the osmotic pressure increased with the increase in the concentration, but the osmotic pressure showed concentration dependency different from that of the carrier material of the present invention, that is, there was almost no dependency on the number of branches, and the osmotic pressure decreased as the molecular weight increased. In general, it has been found that the osmotic pressure of the polymer solution can be approximated by the following expression (*) regardless of the molecular weight of the polymer, the type of the main chain, and the solvent as described above.

[Expression 7]

$$\Pi_0 = \frac{M}{cRT}\{1 + c/c^* + 0.25(c/c^*)^2\}$$

Also, for the overlapping concentration c*, values of c* (10 k) = 59 g/L, c* (20 k) = 34 g/L, and c* (40 k) = 19 g/L are taken.

In order to normalize the effect of the shape of the polymer, the results obtained by replacing the vertical axis and the horizontal axis with the following parameters are shown in Fig. 3.

[Expression 8]

$$\hat{\Pi} = \Pi M/(cRT)$$

[Expression 9]

$$\hat{c} = c/c^*$$

It has been found that the osmotic pressure of the polymer solution of Comparative Example follows the expression (*) indicated by the solid line in Fig. 3, whereas the carrier material of the present invention agrees with the expression (*) in the region of c/c* > 3 or so, but the osmotic pressure is lower than the theoretical value of the expression (*) at concentrations below the c/c* > 3, and as described above, the osmotic pressure of the polymer solution of Comparative Example follows the following expression:

[Expression 10]

$$\Pi = 1.71c^{2.31}$$

Also, the results of viscosity measurement of di-branched PEG (10 k) depending on the presence or absence of associative property are shown in the following table. As the viscosity, the zero-shear viscosity is used. When the viscosity of the solution containing the carrier material of the present invention was compared with the viscosity of a polymer having

the same polymer backbone (having no associative functional group), it has been found that the viscosity does not decrease by having an associative functional group, and the viscosity is non-inferior as compared with the existing polymer solution formulation.

[Table 1]

| | | | |
|---|---|---|---|
| Di-branch (10 k) | | | |
| Concentration (g/L) | 40 | 60 | 80 |
| Without associative group (Pa · s) | 0.0037 | 0.0067 | 0.0092 |
| With associative group (Pa · s) | 0.0052: | 0.018 | 0.025 |

3. Design and Characterization of PEG Carrier: Comparison of Hydrogel, Sponge, and Slime

A carrier material (i) of the present invention was prepared using the polymer unit synthesized in the above 1. (Fig. 1). For comparison, PEG-derived carriers of (ii) and (iii) that differ in crosslinking strategy and pore size were used (Fig. 2).

(i) PEG slime; A viscoelastic polymer liquid obtained by mixing Tetra-PEG-GDL and Tetra-PEG-FPBA to form reversible and variable crosslinks of cyclic ester bonds. It corresponds to the carrier material of the present invention;
(ii) PEG hydrogel; Synthetic hydrogel as artificial extracellular matrix (ECM) cross-linked with Tetra-PEG-PA and Tetra-PEG-GS to form amide bonds and introduced ester bonds as hydrolysable segments of hydrogel;
(iii) PEG sponge; A porous hydrogel obtained by introducing a phase separation structure into a PEG hydrogel obtained by adding potassium sulfate to a polymer unit solution.

These PEG carriers have different features and structures. Differences in mechanical characteristics are observed macroscopically upon compression. It was shown that the PEG hydrogel and PEG sponge were fully elastically deformed, whereas the PEG slime gradually lost its original shape over time. In overall observations, a PEG slime prepared in a spherical shape could maintain the shape like a solid when grasped with tweezers. However, the grasped slime dropped from the tweezers like a highly viscous liquid and eventually became a flat shape on the table (Fig. 3). The difference between these carriers was quantified by a stress relaxation test observing the time dependence of Young's modulus (E(t)) after applying step strain to the carrier. That is, here, with the lapse of time, E(t) drastically reduced only in the PEG slime, and fell below the measurement limit at about 10 **s.** On the other hand, E(t) slightly reduced in the PEG sponge by water extraction, whereas E(t) reached a constant finite value of E(t) after 10 s in the PEG sponge PEG hydrogel (Fig. 4).

Next, structural differences were evaluated by microscopic observation of PEG carriers prepared with red fluorescently labeled PEG precursors (the terminal moiety of the PEG precursors was partly modified with red fluorescent dye). In the PEG sponge, a sea-island structure in which black cavities were dispersed was observed in the image, and the pore size was on the order of 10 μm, whereas in the PEG hydrogel and slime, no characteristic structure was observed, suggesting a network structure on the order of nm (Fig. 5). Phase separation is induced by the addition of an inorganic salt, which reduces the solubility of PEG, resulting in opaque hydrogels. During the gelation process, the PEG precursors bind to each other to increase the molecular weight and promote phase separation. The salt concentration was appropriately set so as to induce phase separation during the gelation, not to induce phase separation of the original precursor solutions.

In vitro evaluation of PEG carrier characteristics.

To investigate the differences in physical characteristics between the PEG carriers in detail, in vitro comparative analysis were further performed. An experimental model was designed to facilitate understanding of the dissolution kinetics. Red fluorescently labeled PEG carriers were prepared on culture inserts with 8.0 um pore size (smaller materials could pass through). For a detailed evaluation of PEG slimes in particular, another PEG slime that was designed to dissolve faster than the standard PEG slime (with a molar ratio of di- and tetra-branched polymers of 5:95, hereinafter referred to as "PEG soft slime") was prepared. The dissolution rate, calculated by measuring the fluorescence intensity of the eluted samples passed through the insert at predetermined time intervals, was gradually increased for each scaffold, and the dissolution rates at 24 hours ($D_{sample}$) were $D_{hydrogel}$ = 19%, $D_{sponge}$ = 16%, $D_{slime}$ = 86%, and $D_{soft\text{-}slime}$ = 90% (Fig. 2b). Furthermore, fluorescent nanoparticles with a diameter of 30 nm were encapsulated as model materials similar in diameter to AAVs, and the release profile of the fluorescent nanoparticles from the PEG carriers was calculated. The release behavior of the particles encapsulated in each carrier showed a similar trend to the dissolution behavior, and the release rates at 24 hours ($R_{sample}$) were $R_{hydrogel}$ = 38%, $D_{sponge}$ = 33%, $D_{slime}$ = 75%, and $D_{soft\text{-}slime}$ = 92%, indicating that the release of nanoparticles was governed by the dissolution of the carriers (Fig. 6). Plots of the dissolution rates against the release rates (Fig. 7) were identical in the PEG slimes. This strong correlation demonstrates that the release of the particles is governed by the dissolution of the PEG slimes. This result suggests that the PEG slimes are thermodynamically

liquids and transport particle diffusion while eluting outside the membrane (inside the body).

In particular, the initial release rates per unit time were almost constant for 7 hours, that is, an ideal zero-order release kinetics were achieved. This result is considered to be due to the fact that the elution, which governs the release of the particles, is approximated as zero-order as it occurs only through the membrane. On the other hand, the release of the particles from the PEG hydrogels and sponges did not correlate with the region of a high release rate per unit time. This is because these carriers are solid and the particles are released mainly by diffusion.

Efficiency of AAV gene transduction in skin ulcers

For the development of therapeutic agents, gene transduction methods that are highly specific to the surface of ulcers are preferred. To verify the influence of the PEG carriers on gene transduction with an AAV on skin ulcers, skin ulcers were generated on the back of mice and a skin chamber model fitted with a sterile silicon chamber was used. GFPNLS-AAVDJ (a type of AAV optimized for cells in skin ulcers encoding GFP with a nuclear localization signal (NLS)) was mixed with the PEG carriers and inoculated onto ulcers (Fig. 8). Considering the transgene expression time by AAV and cell turnover, a 72 hour interval was adopted as the experimental timeframe for the following quantitative analysis. Upon observation of the ulcer surfaces with a fluorescence stereomicroscope, GFPNLS positive cells were observed more frequently in animals treated with AAVs encapsulated in PEG slime, PEG soft slime, or PBS than in animals treated with PEG hydrogel and PEG sponge (Figs. 9 and 10).

Superficial layer-specific AAV gene transduction on skin ulcers

Encapsulation with PEG hydrogel and PEG sponge reduced the efficiency of gene transduction to the surface of skin ulcers, whereas encapsulation with PEG slime retained the original strength observed with PBS. This revealed that PEG slime is preferred for further histological evaluation, with emphasis on the gene transduction specificity in the superficial layer of skin ulcers. Ulcers treated with GFPNLS-AAVDJs and PEG slime, PEG soft slime, and PBS were prepared, and the frequency of the number of GFPNLS-positive cells in each of the superficial layer (granulation tissue and adipose tissue) and the deep layer (fascia and muscle) was histologically analyzed. In agreement with the results of the stereoscopic evaluation, there was no significant difference in the superficial layers of animals treated with PEG slime, PEG soft slime, or PBS. In contrast, the number of GFPNLS positive cells in the deep layer tissue significantly reduced in animals using PEG slime and soft slime, and the degree of reduction was greater in animals using PEG slime (Figs. 11 and 12). Since it was shown that the local layer-specific efficiency of the gene transduction was improved when a PEG slime carrier was used, the influence on distant off-target effects was further investigated.

Reduction of distant off-target gene expression in liver

Like most of AAVs, the distant off-target effects of an AAVDJ vector are concentrated to the liver. To investigate the influence of encapsulation with PEG slime, off-target GFPNLS expression in the liver was evaluated 28 days after inoculation of the ulcers using a PEG slime carrier, or without using a PEG slime carrier (that is, in PBS) in a skin chamber. A small number of GFPNLS positive cells were seen on the surface of the liver using a stereomicroscope (Fig. 13). Quantitative comparison of AAV titers with qPCR in the liver indicated that genomic copies were significantly reduced with the use of the PEG slime carrier (Fig. 14). Thus, it was suggested that the use of the PEG slime carrier reduces the distant off-target effects.

Behavior of nanoparticles on surface of skin ulcers

A series of in vivo experiments demonstrated that PEG slimes were useful carriers for surface-specific AAV gene transduction, whereas PEG sponges and PEG hydrogels showed reduced gene transduction efficiency itself. This difference is considered to be related to the release profiles of the PEG slimes, as in the in vitro evaluation. To elucidate possible mechanistic characteristics unique to the PEG slimes, the behavior of nanoparticles on the surface of skin ulcers was evaluated.

In the initial test, green fluorescent nanoparticles (a diameter of about 30 nm) in PBS were inoculated. The nanoparticles were initially localized in the superficial layer, and diffused into the deep layers such as adipose and muscle tissues over time. Importantly, only a few particles remained on the surface of the ulcer at 24 hours. Therefore, 24 hours was adopted as the experimental time frame for the following quantitative analysis of the influence of the PEG carrier on the release of the nanoparticles and the dissolution of PEG.

Green fluorescent nanoparticles were mixed with PEG carriers or PBS and inoculated onto ulcers (Fig. 15). It was found that when the PEG carrier was used, more nanoparticles remained on the superficial layer at 24 hours than the nanoparticles in PBS. This tendency was greater particularly in the PEG slime and soft slime, than in the PEG hydrogel and

PEG sponge (Fig. 16). The extension of the surface-specific distribution of nanoparticles by the PEG slime carrier may be related to surface-specific AAV gene transduction. The in vivo gene transduction efficiency varied depending on the type of carrier PEG. This difference is considered to be attributed to the dissolution mechanism due to the crosslinking form.

Discussion

The PEG hydrogels and PEG sponges, which are comparative examples, have a structure in which all molecules are connected through irreversible bonds. Therefore, they have properties characteristic of solids. In vitro characterization has shown that they exhibit a finite elastic modulus under constant strain (Fig. 4). In vitro evaluation of the PEG dissolution rates per unit time and the particle release rates per unit time showed a significant delay in both rates when hydrogels and sponges were applied. This suggests that the nanoparticles were trapped in the networks (Fig. 6).

In general, solid polymer materials such as PEG hydrogels and sponges are thought to degrade uniformly, which is called bulk degradation, rather than to degrade gradually from the surface. That is, chain scission occurs uniformly over time by hydrolysis. Eventually, when the network connection falls below a certain level, the PEG hydrogels and sponges transition from a solid to a liquid. Therefore, in the PEG hydrogels and sponges after being degraded, AAV particles penetrate into tissues, and thus the amount of particles released is thought to be reduced (Fig. 17a). Indeed, fluorescent particle experiments to evaluate the physical movement of nanoparticles showed that both the hydrogels and the sponges reduced the amount of particles in the shallow ulcer layer (Fig. 17).

For successful infection by endocytosis, the AAV particles require direct contact with cells. Therefore, AAV infection was suppressed while the AAV particles were trapped in a PEG network under the condition that there was no direct contact with cells. In other words, unreleased AAV particles were not infectious, which resulted in reduced GFP expression on the ulcer surface after AAV administration using a PEG hydrogel or sponge.

On the other hand, in the PEG slime carrier of the present invention in an equilibrium state of binding and dissociation, the constituent molecules are connected through reversible bonds. The elastic modulus of the slime reduces and reaches zero over time (Fig. 4). This indicates that the slime is a liquid from a thermodynamic point of view. Therefore, the PEG slime applied to the surface of ulcer diffuses as a liquid and penetrates from the surface of ulcer into deep tissues. PEG slime and PBS were equivalent in terms of carrier and AAV diffusion.

However, the PEG slime showed distinctive features that differed from PBS. The dissolution rate per unit time and release rate per unit time of the slime were both lower than those of PBS (Fig. 6), and the dissolution rate per unit time and release rate per unit time were almost the same (Fig. 7). These results indicate that the particles were released at the same time as the local dissociation of PEG itself in the slime. Indeed, the nanoparticles contained in the PEG slime were confined to the ulcer surface after application (Fig. 16), limiting penetration from the stroma into the deep tissue. That is, unlike the solid-like PEG hydrogels and sponges, the particles in the liquid-like PEG slime were translationally diffused, and AAV infection occurred mainly on the ulcer surface where the PEG slime was present (Fig. 17b).

Furthermore, the PEG slime was more localized upon viral infection than the highly viscoelastic PEG soft slime, resulting in mainly reduced deep infection (Fig. 12). Since the local off-target deep layer infections were limited, expression at distant off-targets was also reduced in the liver.

## Claims

1. A carrier material for locally releasingparticles, wherein the carrier material has a finite zero-shear viscosity higher than 500 Pa · **s,**

   contains a non-gelling polymer formed by reversibly bonding a plurality of hydrophilic polymer units, and contains a solvent,
   the polymer units include a first branched polymer unit having a total of two or more boronic acid-containing groups on side chains or terminals, and a second branched polymer unit having a total of two or more diol groups on side chains or terminals, and
   a polymer concentration (c) in the carrier material is greater than or equal to an overlapping concentration (c*) of the polymer units.

2. The carrier material according to claim 1, wherein the particles have a diameter in the range of 10 to 100 nm.

3. The carrier material according to claim 1, wherein the particles include is an adeno-associated virus (AAV).

4. The carrier material according to claim 1, wherein the polymer unit has a polyethylene glycol backbone or a polyvinyl backbone.

**5.** The carrier material according to claim 1, wherein the first branched polymer unit is di-, tri-, or tetra-branched polyethylene glycol.

**6.** The carrier material according to claim 1, wherein the second branched polymer unit is di-, tri-, or tetra-branched polyethylene glycol, or di-branched polyvinyl alcohol.

**7.** The carrier material according to claim 1, wherein the first branched polymer unit has a molecular weight (Mw) of $5 \times 10^3$ to $1 \times 10^5$.

**8.** The carrier material according to claim 1, wherein the boronic acid-containing group is an arylboronic acid optionally substituted with a halogen atom.

**9.** The carrier material according to claim 1, wherein the diol group has a ring-opened structure of a sugar derivative.

**10.** The carrier material according to claim 1, further comprising a third polymer unit having a total of two or more boronic acid-containing groups or diol groups on side chains or terminals.

**11.** The carrier material according to claim 1, wherein an osmotic pressure of the carrier material is less than an osmotic pressure $\Pi_0$ (Pa) expressed by the following expression:

[Expression 1]

$$\Pi_0 = \frac{M}{cRT}\{1 + c/c^* + 0.25(c/c^*)^2\}$$

wherein c represents a polymer concentration (g/L), c* represents an overlapping concentration of the polymer units, R represents gas constant, T represents an absolute temperature, and M is a weight average molecular weight of the polymer units.

**12.** The carrier material according to claim 1, wherein the carrier material has an ability to release the particles in vivo along with elution of the carrier material, and a dissolution rate L and a release rate M at that time satisfy a relationship of:

[Expression 2]

$$L = A \times M$$

wherein A is in a range of $0.9 < A < 1.1$.

**13.** The carrier material according to claim 1, wherein an application of the locally releasing is a gene therapy for a skin ulcer.

**14.** A composition comprising the carrier material according to any one of claims 1 to 13 and the particles stored in the carrier material.

**15.** A treatment method comprising applying the composition according to claim 14 to an affected part.

**16.** A kit for forming the carrier material according to any one of claims 1 to 13, comprising at least a composition A containing a first branched polymer unit having a total of two or more boronic acid-containing groups on side chains or terminals, and a composition B containing a second branched polymer unit having a total of two or more diol groups on side chains or terminals, wherein the carrier material has a finite zero-shear viscosity of greater than 500 Pa · s, and a polymer content (c) in the carrier material is greater than or equal to an overlapping concentration (c*) of the polymer units.

**17.** The kit according to claim 16, further comprising a polymer solution C containing a third polymer unit having a total of two or more boronic acid-containing groups or diol groups on side chains or terminals.

# *FIG. 1*

**PEG slime**

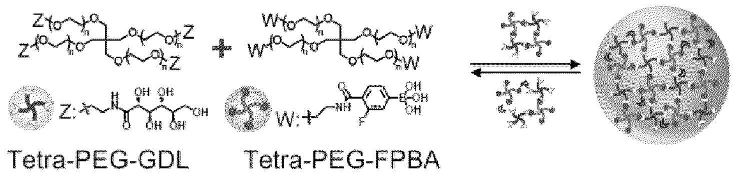

Tetra-PEG-GDL Tetra-PEG-FPBA

# *FIG. 2*

**PEG hydrogel**

**PEG sponge**

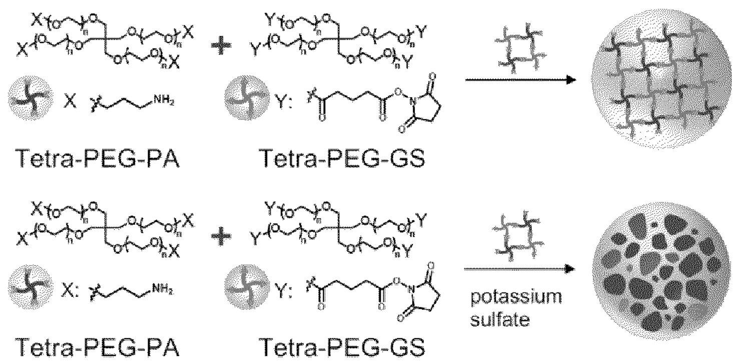


Tetra-PEG-PA Tetra-PEG-GS

Tetra-PEG-PA Tetra-PEG-GS

FIG. 3

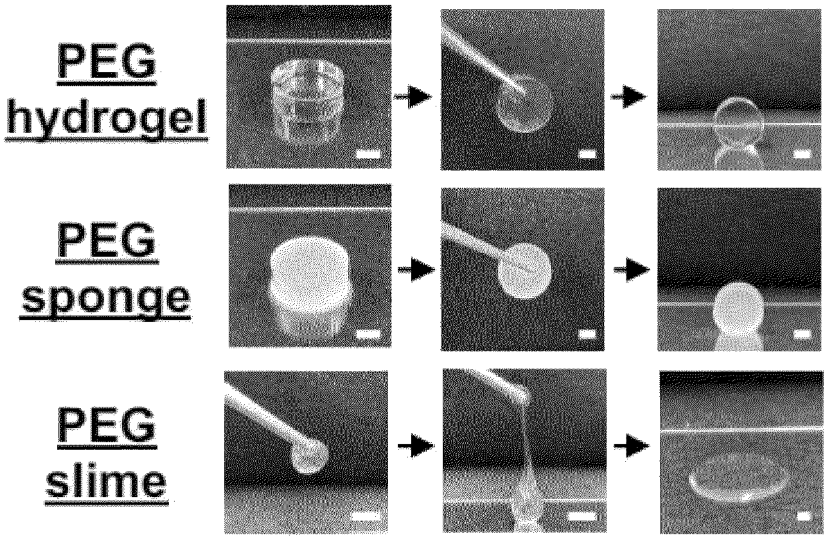

# FIG. 4

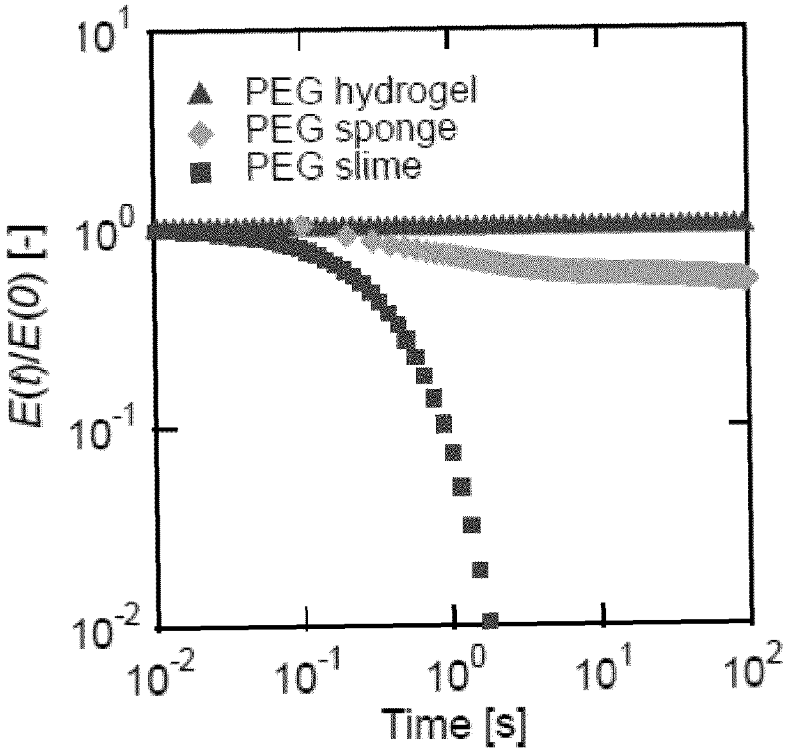
PEG hydrogel
PEG sponge
PEG slime
$10^1$
$10^0$
$10^{-1}$
$10^{-2}$
E(t)/E(0) [-]
$10^{-2}$
$10^{-1}$
$10^0$
$10^1$
$10^2$
Time [s]

FIG. 5

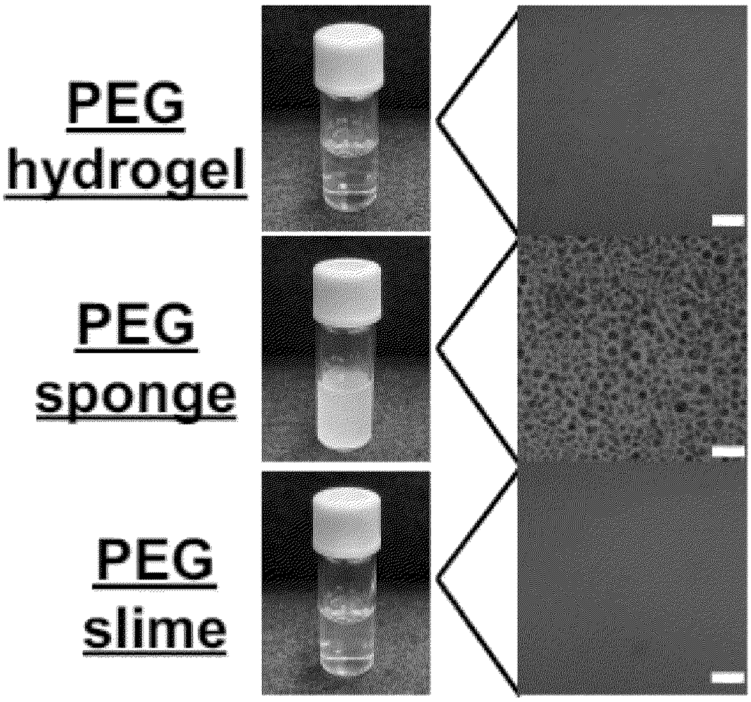

FIG. 6

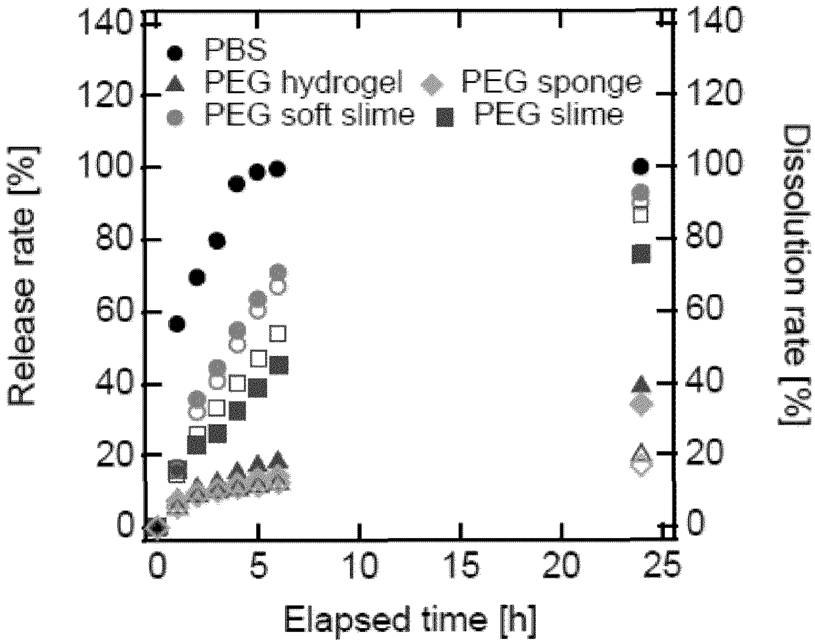
PBS
PEG hydrogel
PEG sponge
PEG soft slime
PEG slime
Release rate [%]
Dissolution rate [%]
Elapsed time [h]
0
20
40
60
80
100
120
140
0
5
10
15
20
25

FIG. 7

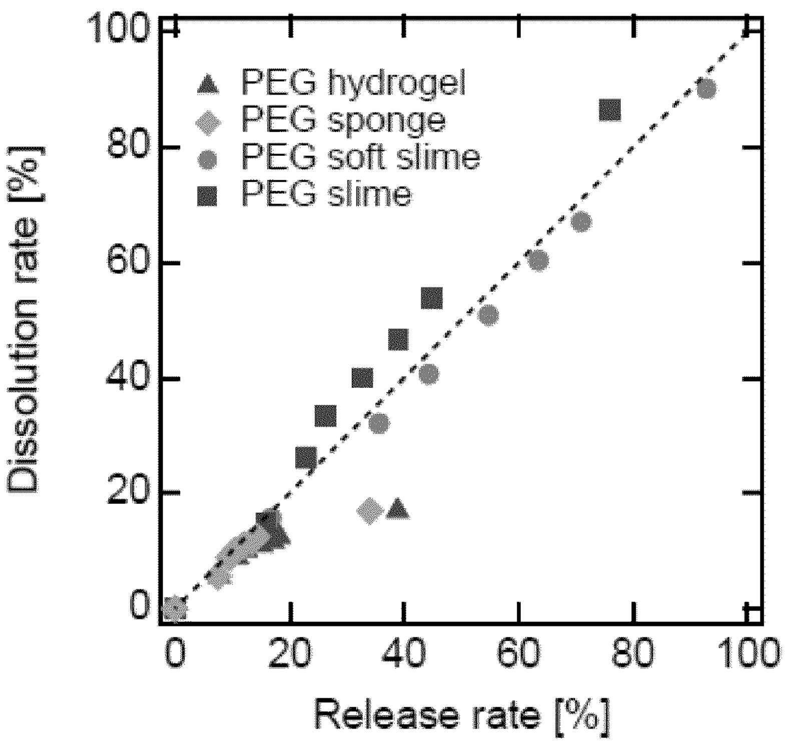
PEG hydrogel
PEG sponge
PEG soft slime
PEG slime
Dissolution rate [%]
Release rate [%]
0
20
40
60
80
100

*FIG. 8*

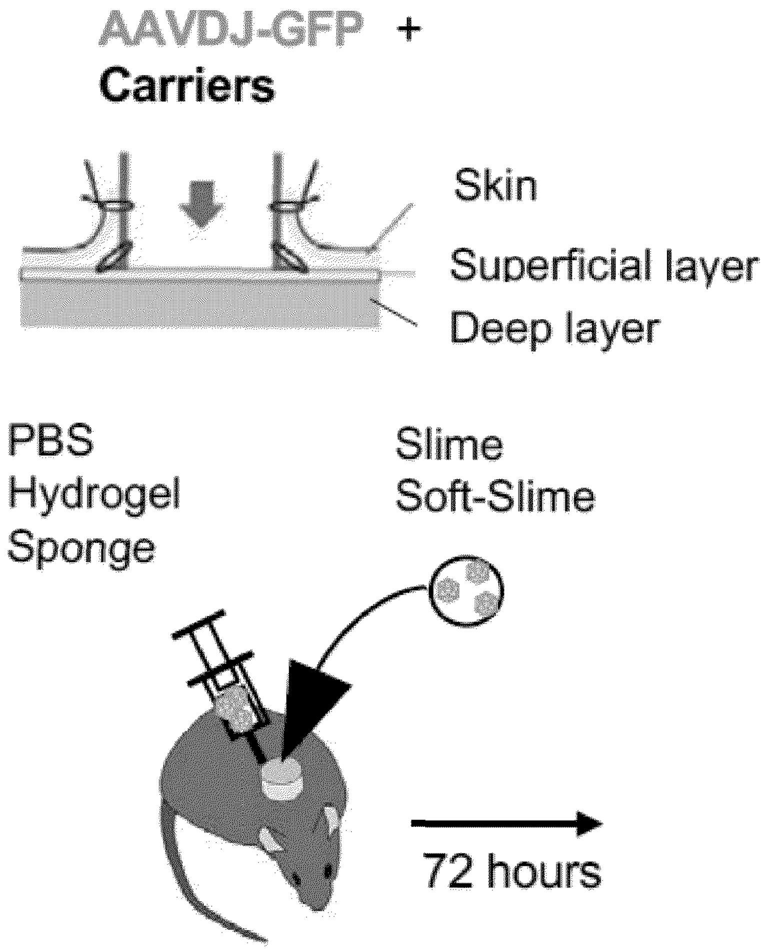
AAVDJ-GFP +
Carriers
Skin
Superficial layer
Deep layer
PBS
Hydrogel
Sponge
Slime
Soft-Slime
72 hours

FIG. 9

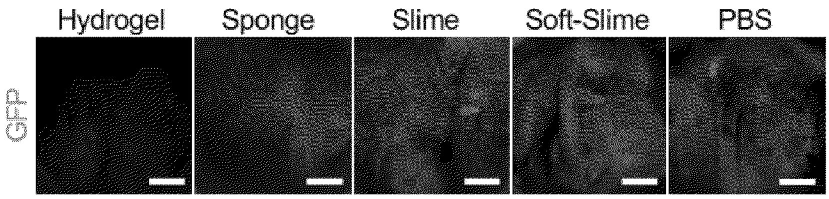
Hydrogel
Sponge
Slime
Soft-Slime
PBS
GFP

FIG. 10

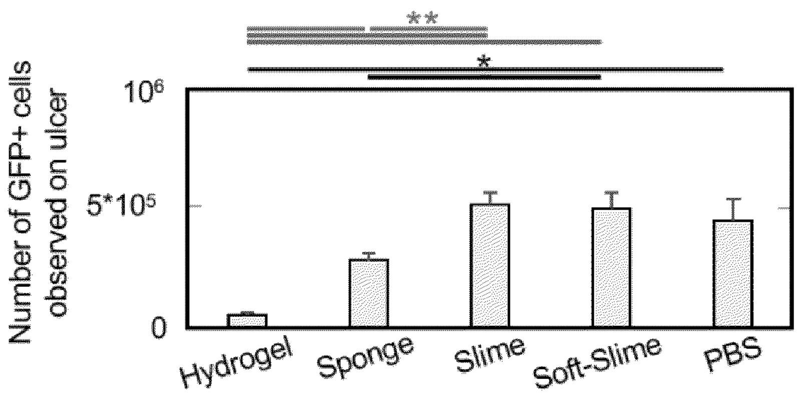
**
*
Number of GFP+ cells observed on ulcer
10^6
5*10^5
0
Hydrogel
Sponge
Slime
Soft-Slime
PBS

FIG. 11

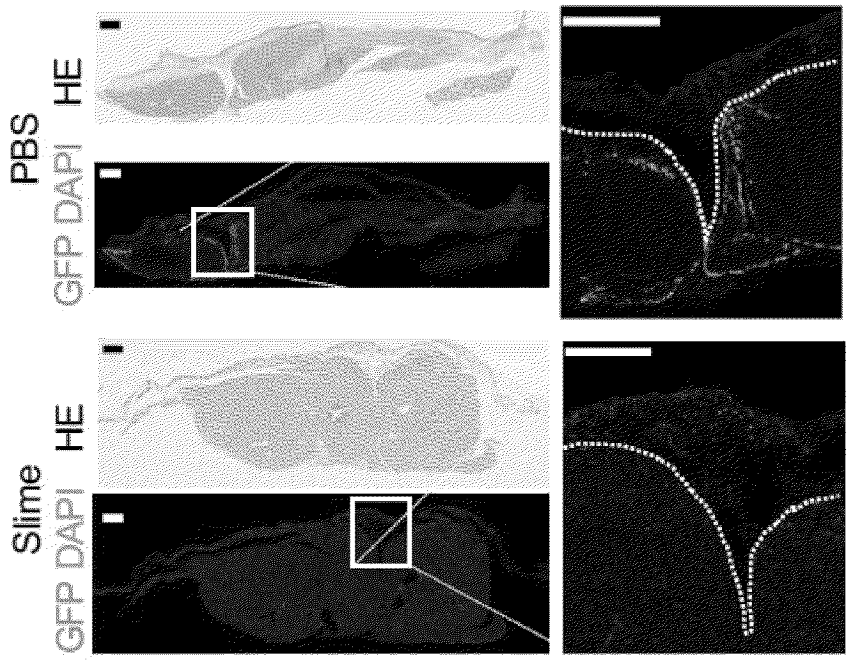

FIG. 12

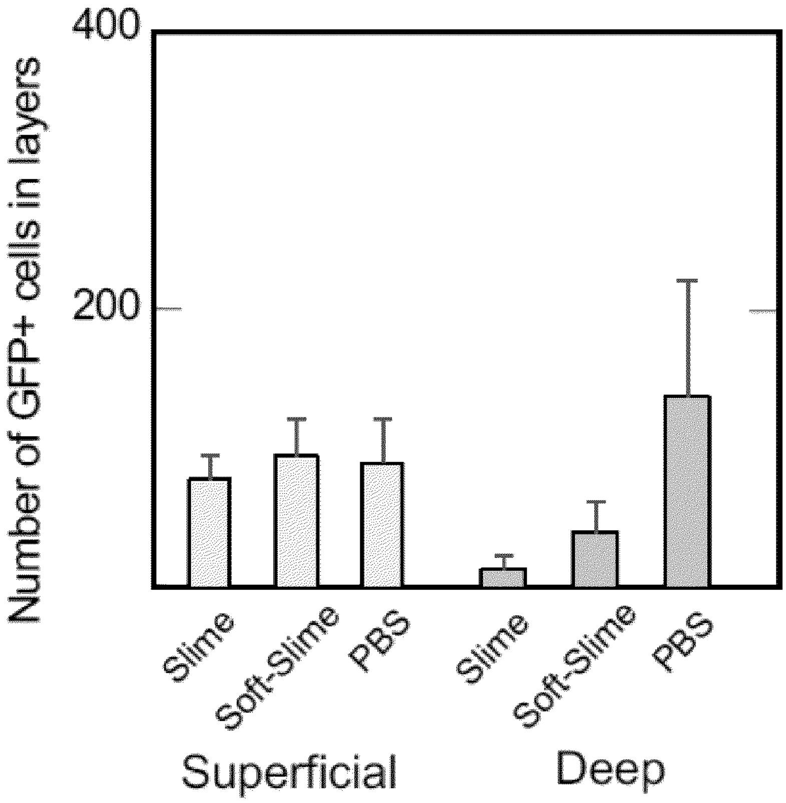
400
200
Number of GFP+ cells in layers
Slime
Soft-Slime
PBS
Slime
Soft-Slime
PBS
Superficial
Deep

FIG. 13

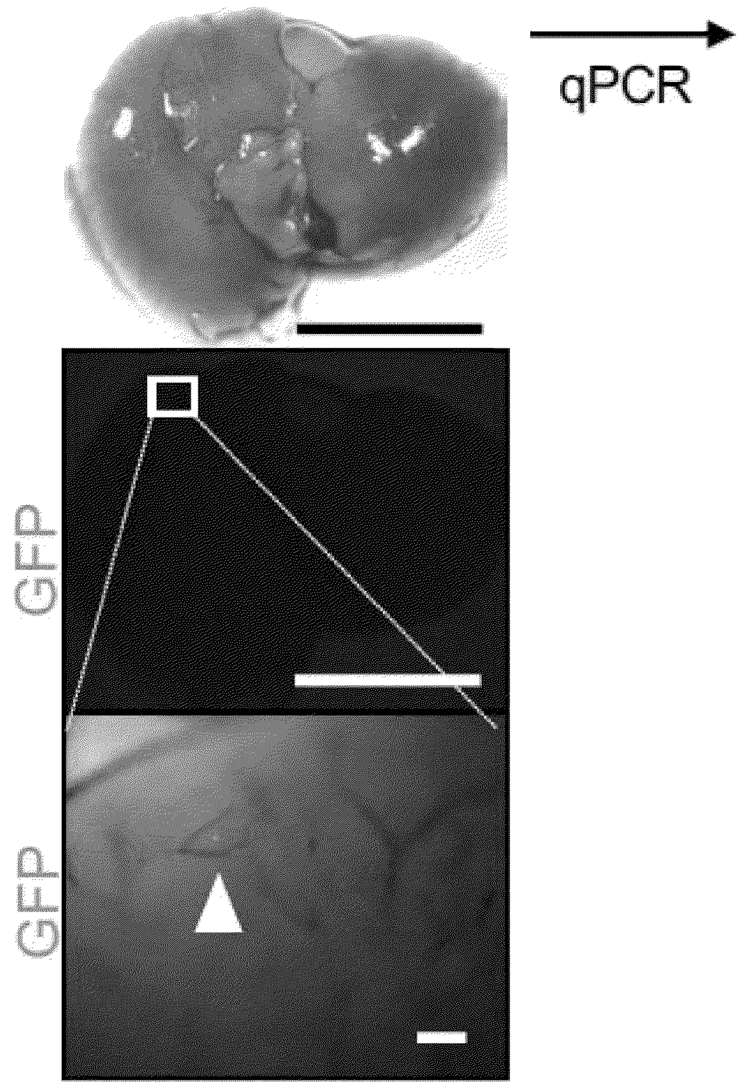

*FIG. 14*

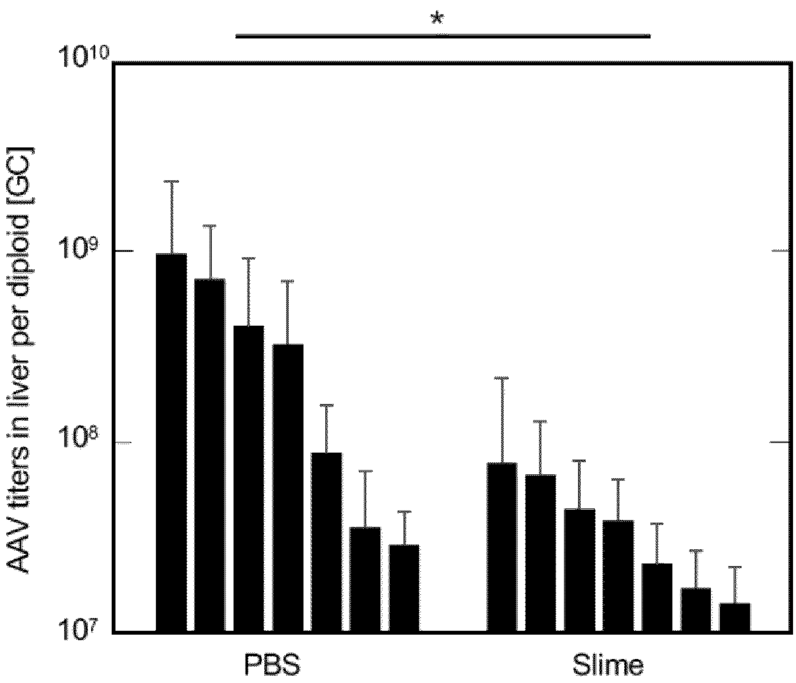
*
$10^{10}$
$10^{9}$
$10^{8}$
$10^{7}$
AAV titers in liver per diploid [GC]
PBS
Slime

*FIG. 15*

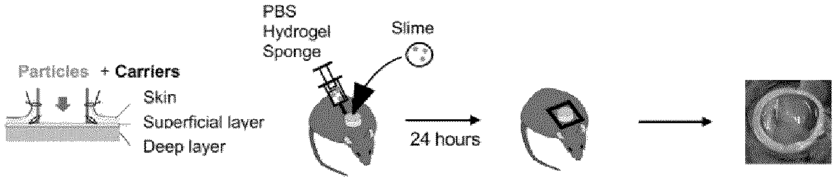
PBS
Hydrogel
Sponge
Slime
Particles + Carriers
Skin
Superficial layer
Deep layer
24 hours

## FIG. 16

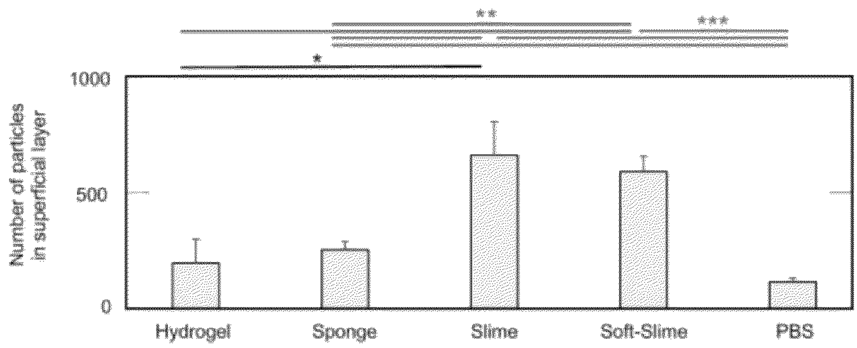
**
***
*
1000
500
0
Number of particles in superficial layer
Hydrogel
Sponge
Slime
Soft-Slime
PBS

## FIG. 17

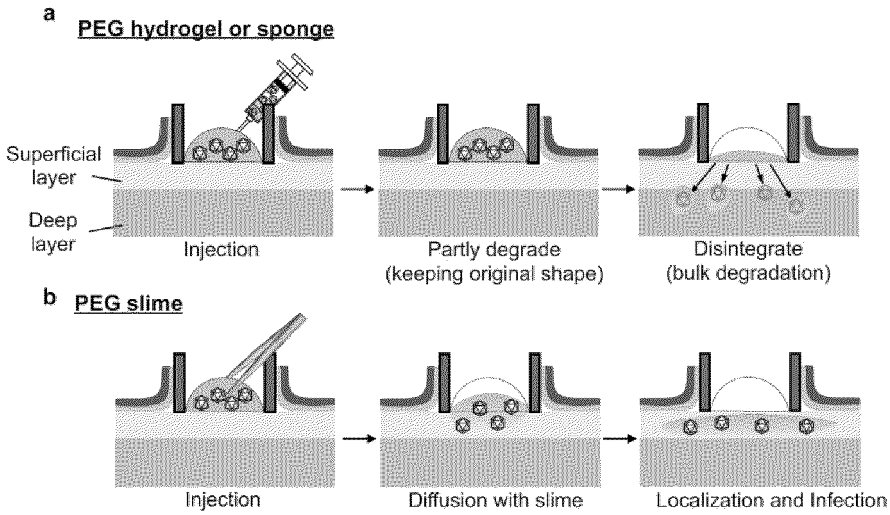
a
PEG hydrogel or sponge
Superficial layer
Deep layer
Injection
Partly degrade (keeping original shape)
Disintegrate (bulk degradation)
b
PEG slime
Injection
Diffusion with slime
Localization and Infection

**INTERNATIONAL SEARCH REPORT**

International application No. **PCT/JP2023/037744**

**A. CLASSIFICATION OF SUBJECT MATTER**

***A61K 47/34***(2017.01)i; ***A61K 47/32***(2006.01)i; ***A61K 48/00***(2006.01)i; ***A61P 17/02***(2006.01)i
FI: A61K47/34; A61K47/32; A61K48/00; A61P17/02

According to International Patent Classification (IPC) or to both national classification and IPC

**B. FIELDS SEARCHED**

Minimum documentation searched (classification system followed by classification symbols)

A61K47/34; A61K47/32; A61K48/00; A61P17/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2022/092043 A1 (UNIV TOKYO) 05 May 2022 (2022-05-05) claims 1-11, paragraphs [0002], [0050]-[0062] | 1-17 |
| A | WO 2015/170757 A1 (UNIV TOKYO) 12 November 2015 (2015-11-12) paragraphs [0059], [0063]-[0094] | 1-17 |
| A | JP 2012-500208 A (CALIFORNIA INSTITUTE OF TECHNOLOGY) 05 January 2012 (2012-01-05) claim 24, paragraph [0184] | 1-17 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 January 2024** | **23 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2023/037744**

| Patent document cited in search report | Publication date (day/month/year) | Patent family member(s) | Publication date (day/month/year) |
|---|---|---|---|
| WO 2022/092043 A1 | 05 May 2022 | (Family: none) | |
| WO 2015/170757 A1 | 12 November 2015 | US 2021/0187005 A1<br>paragraphs [0133]-[0160]<br>EP 3141243 A1<br>paragraphs [0064], [0066]-[0097] | |
| JP 2012-500208 A | 05 January 2012 | US 2010/0040556 A1<br>paragraphs [0226]-[0231], claim 24<br>WO 2010/019718 A2<br>paragraphs [0199]-[0205], claim 24 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007083522 A


### Non-patent literature cited in the description


- **KURITA, M** ; **IZPISUA BELMONTE, J. C** ; **SUZUKI, K** ; **OKAZAKI, M.** *J Dermatol Sci*, 2019, vol. 95, 8-12
- **KURITA, M** ; **ARAOKA, T** ; **HISHIDA, T. et al.** *Nature*, 2018, vol. 561, 243-247
- **MATSUNAGA et al.** *Macromolecules*, 2009, vol. 42 (4), 1344-1351
- **FUJIYABU et al.** *Science Advances*, 2022, vol. 8, eabk0010